(19) **Europäisches Patentamt / European Patent Office / Office européen des brevets**

(11) **EP 2 796 856 B1**

(12) # EUROPEAN PATENT SPECIFICATION

(45) Date of publication and mention
of the grant of the patent:
**01.01.2020 Bulletin 2020/01**

(21) Application number: **12860685.2**

(22) Date of filing: **30.08.2012**

(51) Int Cl.:
*G01N 21/3504* (2014.01) *G01N 7/18* (2006.01)
*G01N 33/22* (2006.01)

(86) International application number:
**PCT/CN2012/080795**

(87) International publication number:
**WO 2013/091399 (27.06.2013 Gazette 2013/26)**

(54) **COAL GAS COMPONENT AND CALORIFIC VALUE MEASUREMENT METHOD**

KOHLEGASKOMPONENTE UND HEIZWERTMESSVERFAHREN

PROCÉDÉ DE MESURE DE COMPOSANTS ET DE POUVOIR CALORIFIQUE DE GAZ DE HOUILLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2011 CN 201110435862**

(43) Date of publication of application:
**29.10.2014 Bulletin 2014/44**

(73) Proprietor: **Wuhan Cubic Optoelectronics Co. Ltd.
Hubei 430205 (CN)**

(72) Inventors:
• **LIU, Zhiqiang**
  **Wuhan, Hubei 430205 (CN)**
• **XIONG, Youhui**
  **Wuhan, Hubei 430205 (CN)**
• **HE, Tao**
  **Wuhan, Hubei 430205 (CN)**

• **SHI, Pingjing**
  **Wuhan, Hubei 430205 (CN)**

(74) Representative: **Zeuner Summerer Stütz
Patent- und Rechtsanwälte
Partnerschaft
Nußbaumstraße 8
80336 München (DE)**

(56) References cited:
**WO-A1-2011/155086     CN-A- 1 544 949
CN-A- 102 539 374     CN-Y- 2 812 004
CN-Y- 2 874 488     CN-Y- 200 947 084
DE-A1-102005 005 727     DE-B3-102008 029 553
DE-B3-102008 038 278     JP-A- H08 320 300
JP-A- H11 173 989     JP-A- 2004 325 458
US-A1- 2002 124 630**

**Description**

FIELD OF THE INVENTION

The invention relates to a method for measuring the coal gas component and calorific value, and more particularly to a coal gas component and calorific value measurement method.

BACKGROUND OF THE INVENTION

Coal gasification is an important part of the coal chemical industry in China, and especially appears more important under the conditions that China is increasingly short of oil resources. Detection analysis of the coal gas component is the precondition for gasifier optimization control, and is also an important parameter of other processes in the coal chemical industry. It is also usually necessary to detect the coal gas component in the industrial fields, such as blast furnace, converter, coke oven, glass and ceramics.

As a classical manual chemical analyzer, Orsat gas analyzer is characterized by low price, easy operation and easy maintenance etc., and has always been widely used in the field of coal gas component analysis. However, this method suffers from manual operation, low accuracy and low speed, and can be applicable to the needs of industrial development no longer. In recent years, the chromatograph has been promoted widely, but it needs to test the gas after separation with a plurality of chromatographic columns in the presence of a carrier gas, which is difficult to realize real-time online test. Infrared gas analyzer has been used in China for years, but the former technology usually can only be used to analyze a single or two components with a set of analytical instrument. And, it suffers from not only a stiff price but also complex maintenance. In addition, it is impossible to accurately measure $CH_4$ in the coal gas due to the presence of other hydrocarbons and mutual interference thereof. The volume concentration of $H_2$ and $O_2$ in the coal gas cannot be measured with NDIR method, that of $H_2$ is usually measured with TCD, and that of $O_2$ is measured with ECD. Besides, $CH_4$ and other $C_nH_m$ in the coal gas interfere with each other, and $CH_4$ and $CO_2$ etc. will interfere with $H_2$. In the patent CN201886002U, the inventor has developed a coal gas analysis system, which can achieve the purpose of analyzing gas concentration, such as $CO_2$, $O_2$, CO, $CH_4$, $H_2$, $C_2H_4$ and $C_2H_6$, in the coal gas with chromatographic analysis technique through one-time sample injection, but this system needs to analyze the coal gas component in the presence of a carrier gas, can only achieve intermittent measurement, and cannot achieve site measurement. Besides, restricted by the chromatographic column, it is impossible to measure $C_3H_8$ and $C_4H_{10}$ etc., so it is impossible to get accurate calorific value of the coal gas. In the patent CN101750439A, the inventor has provided an improved single-channel hydrogen detector, which minimizes the baseline drift caused by the environmental change and its temperature change through effectively improving and optimizing the physical structure of hydrogen detector based on the principle of thermal conductivity, but is calibrated using $H_2$ in $N_2$. If other components other than $H_2$ in the coal gas change, especially after $CO_2$ and $CH_4$ change, accurate concentration can only be obtained after revising the hydrogen concentration. Due the absence of $CO_2$ and $CH_4$ data, it is impossible to complete revision. In the patent US2008011952A1, ABB (Asea Brown Boveri Ltd) company proposes a non-dispersive infrared gas analyzer, which can set up a plurality of gas sensors using the non-dispersive infrared technology, and can achieve the purpose of measuring a variety of asymmetric gas concentrations. Besides, each gas detector has a measuring channel and a comparison channel, so as to improve the measurement accuracy. But such analyzer usually measures $CH_4$ and $C_nH_m$ using a narrowband filter of about 3.4 um. In the context of gas measurement, due to very significant mutual interference of $C_nH_m$ and $CH_4$, it is impossible to measure $CH_4$ and $C_nH_m$ in the main source of calorific value of coal gas. In addition, the instrument cannot be provided with TCD to measure the hydrogen concentration, and even if independently installed instrument of such type is used as the combination instrument, it is more impossible to directly get the calorific value of coal gas because of no revision of $H_2$ measurement result according to $CO_2$ and $CH_4$ etc.

In conclusion, at present, it is impossible to accurately measure various components in the coal gas with only one instrument in China, especially $CH_4$, $C_nH_m$, CO and $H_2$ contributing to energy. Therefore, it is necessary to develop a method to not only measure a variety of components in the coal gas, but also calculate the calorific value of coal gas, and effectively eliminate the interference between gases.

Document DE 10 2005 005 727 A1 discloses a device and method for an online-determination of a gas composition and gas properties of a fuel gas. Document DE 10 2008 029 553 B3 relates to a method for determining the gas quality of an at least partially offset with biogas sample gas.

SUMMARY OF THE INVENTION

In order to overcome the problems and defects of prior art, the invention provides a method for more accurately measuring the coal gas component and calorific value. Compared with the above existing test methods, this method has following advantages: the invention can simultaneously measure a variety of gas components, reduce the interference between different gases by optimizing NDIR narrowband filter parameters, measure $C_nH_m$ with the filter at 3.46 $\mu$m wavelength, convert other hydrocarbons into $C_3H_8$, and facilitate calculating the calorific value of coal gas according to the obtained volume concentration of gas. The manufacturing cost of the analytical instrument in this method is 1/3 as much as that of the calorimeter in the traditional combustion method, and only 1/10 as much as that of the mass spectrum analyzer. Its analysis speed is 30 times more than that of the conventional chromatograph.

Technical scheme of the invention is as follows:

The invention provides the methods of claims 1 and 2.

$CH_4$ is measured using the principle of NDIR; and a selected narrowband filter parameter, that is, a center wavelength (CWL)/half-peak bandwidth (HWBP), is 7.85$\pm$0.05$\mu$m/180$\pm$5 nm.

$C_nH_m$ is measured using the principle of NDIR; and a selected narrowband filter parameter, that is, a CWL/HWBP, is 3.46 $\pm$ 0.05$\mu$m / 120 $\pm$ 5 nm.

CO is measured using the principle of NDIR; and a selected narrowband filter parameter, that is, a CWL/HWBP, is 4.66 $\pm$ 0.05$\mu$m / 90 $\pm$ 5 nm.

$CO_2$ is measured using the principle of NDIR; and a selected narrowband filter parameter, that is, a CWL/HWBP, is 4.26 $\pm$ 0.05$\mu$m / 120 $\pm$ 5 nm.

Compared with the existing test methods, the method of the invention has the following advantages: the invention can simultaneously measure a variety of gas components, reduce the interference between different gases by optimizing NDIR narrowband filter parameters, measure $C_nH_m$ with the filter of 3.46 $\mu$m wavelength, convert other hydrocarbons into $C_3H_8$, and facilitate calculating the calorific value of coal gas according to the obtained volume concentration of gas. The manufacturing cost of the analytical instrument in this method is 1/3 as much as that of the calorimeter in the traditional combustion method, and only 1/10 as much as that of the mass spectrum analyzer. Its analysis speed is 30 times more than that of the conventional chromatograph

BRIEF DESCRIPTION OF THE DRAWINGS

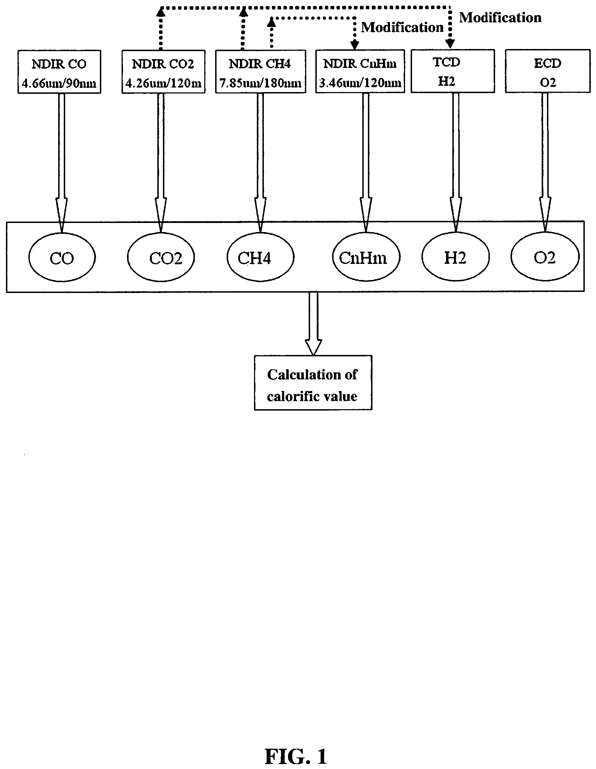

FIG. **1** is a principle diagram for analysis of the coal gas component;

FIG. **2** is an IR absorption spectra of CO, $CO_2$ and $CH_4$;

FIG. **3** is a mutual interference pattern of hydrocarbons approximately at 3.3 $\mu$m; and

FIG **4** is an IR absorption spectrum of $CH_4$.

DETAILED DESCRIPTION OF THE EMBODIMENTS

For further illustrating the invention, experiments detailing a method for measuring the coal gas component and calorific value are described hereinbelow combined with examples.

**Example 1 Gas analyzer for 6 components of coal gas from biomass gasification**

1. Select various gas filter parameters, gas chamber length and measuring range

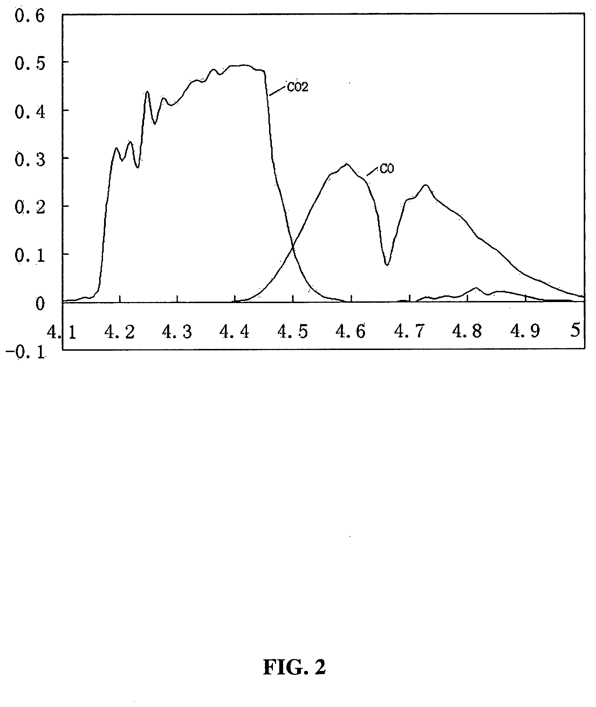

As shown in the IR absorption spectra of CO and $CO_2$ in FIG. **2,** the absorption peak of CO at 4.66 $\mu$m is not affected by $CO_2$, and the absorption peak of $CO_2$ at 4.26 $\mu$m is not affected by CO. Thus, the CO detector with the measuring range of 40% and $CO_2$ detector with the measuring range of 30% are made with the narrowband filter parameters respectively of 4.66$\mu$m/90nm and 4.26$\mu$m/120nm, reference channel of 3.91 $\mu$m, and chamber length respectively of 43 mm and 2 mm.

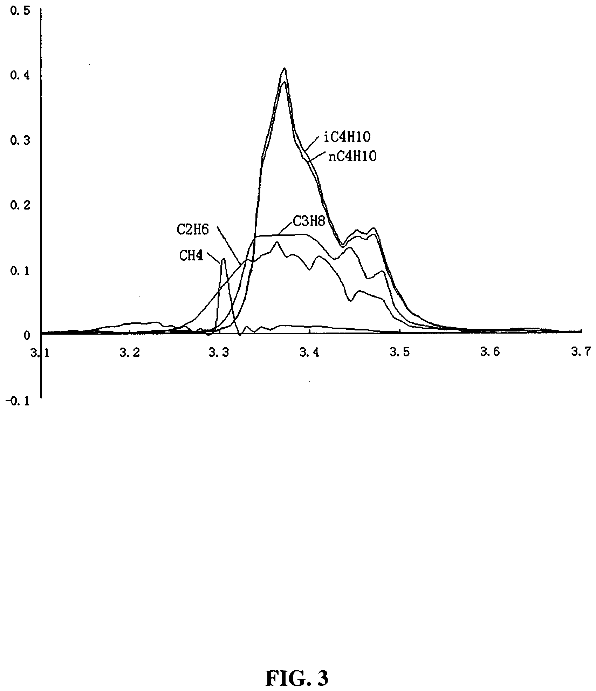

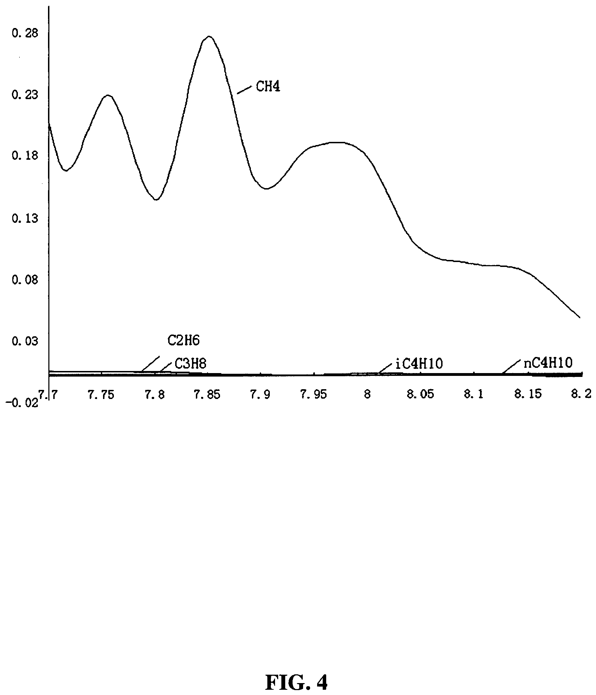

Then, based on the IR absorption spectra of $CH_4$, $C_2H_6$, $C_3H_8$ and $C_4H_{10}$ in FIGS. **3-4,** the absorption peak at 7.85 $\mu$m is selected, instead of the absorption peak at 3.3 $\mu$m, in order to eliminate the influence of $C_nH_m$ on $CH_4$ absorption peak. As a result, the $CH_4$ detector with the measuring range of 20% is made with the narrowband filter parameters of 7.85$\mu$m/180nm, reference channel of 3.91$\mu$m and $CH_4$ chamber of 68 mm long.

According to the IR absorption spectrum of $C_nH_m$ in FIG. **3**, $C_nH_m$ has absorption peaks at 3.3-3.5 $\mu$m. In order to reduce the influence of $CH_4$ on $C_nH_m$, it is necessary to avoid the absorption peak of $CH_4$, and select center wavelength at 3.35-3.5 $\mu$m. Test of different filters at 3.35-3.5 $\mu$m shows that $C_3H_8$ can represent $C_nH_m$ ($C_nH_m$ is calibrated with $C_3H_8$) with the narrowband filter of 3.46 $\mu$m/120 nm as the filter for $C_nH_m$ detector. The $C_nH_m$ detector with the measuring range of 5% is made with the reference channel of 3.91 $\mu$m and $C_nH_m$ chamber of 43 mm long. The test data are provided in Table **1.**

**Table 1 Influence of C1-C5 on the detector at 3.46 $\mu$m**

| Inlet gas (%) | | Volume concentration of $CH_4$ (%) | Volume concentration of $C_nH_m$ (%) | Proportionality coefficient of volume concentration ($C_3H_8/C_nH_m$) | Average proportionality coefficient of volume concentration ($C_3H_8/C_nH_m$) |
|---|---|---|---|---|---|
| $CH_4$ | 0 | 0 | 0 | - | - |
| (C1) | 3.79 | 3.76 | 0.11 | - | - |
| | 7.68 | 7.71 | 0.22 | - | - |
| | 11.67 | 11.66 | 0.34 | - | - |
| | 15.78 | 15.80 | 0.45 | - | - |
| | 20.00 | 20.01 | 0.57 | - | - |
| $C_2H_6$ | 1.01 | 0 | 0.69 | 0.68 | 0.687 |
| (C2) | 2.03 | 0 | 1.41 | 0.69 | |
| | 3.98 | 0 | 2.73 | 0.69 | |
| $C_3H_8$ | 0.43 | 0 | 0.42 | 0.98 | 1.00 |
| (C3) | 1.76 | 0.01 | 1.78 | 1.01 | |
| | 3.42 | 0.01 | 3.44 | 1.01 | |
| | 5.00 | 0.02 | 5.01 | 1.00 | |
| $NC_4$ | 1.01 | 0.01 | 1.32 | 1.31 | 1.315 |
| (C4) | 2.00 | 0.02 | 2.68 | 1.34 | |
| $IC_4$ | 1.01 | 0 | 1.29 | 1.28 | |
| (C4) | 1.99 | 0.01 | 2.64 | 1.33 | |
| $NC_5$ (C5) | 0.998 | 0.01 | 1.67 | 1.67 | 1.68 |
| $IC_5$ (C5) | 1.00 | 0.01 | 1.69 | 1.69 | |

**Table 2 Coefficient of low calorific value of $C_nH_m$**

| Gas name | Coefficient of low calorific value (MJ/m$^3$) | Ratio of coefficient of calorific value ($C_nH_m/C_3H_8$) |
|---|---|---|
| $C_2H_6$ | 64.35 | 0.69 |
| $C_3H_8$ | 93.18 | 1.00 |
| $C_4H_{10}$ | 123.16 | 1.32 |
| $C_5H_{12}$ | 156.63 | 1.68 |

From the comparison of Table 1 and Table 2, the proportionality coefficient of volume concentration of $C_nH_m$ measured with the detector made of narrowband filter at 3.46$\mu$m/120nm is very close to its coefficient of calorific value, so the calorific value of $C_nH_m$ can be calculated as that of $C_3H_8$.

Finally, the known technique is used to make a TCD of $H_2$ with the measuring range of 20% and an ECD of $O_2$ with the measuring range of 25%.

2. Measure the volume concentration of various gases

CO, $CO_2$, $CH_4$ and $C_nH_m$ are measured using NDIR, and the readings are respectively expressed as $T_{CO}$, $T_{CO2}$, $T_{CH4}$ and $T_{CnHm}$.

The volume concentration of $H_2$ is measured with TCD, and is expressed as $T_{H2}$.

The volume concentration of $O_2$ is measured with ECD, and is expressed as $T_{O2}$.

3. Revise the gas volume concentration and calculate the calorific value.

1) Revise the volume concentration of $C_nH_m$ with $CH_4$.

$CH_4$ has certain influence on $C_nH_m$, so it is also necessary to get the revised volume concentration of $C_nH_m$ ($R_{CnHm}$) through revising the measuring result of $C_nH_m$ ($T_{CnHm}$) obtained from the calibration curve according to the volume concentration of $CH_4$ ($T_{CH4}$).

In order to revise the influence of $CH_4$ on the measuring result of $C_nH_m$, the calibrating gas $CH_4$ is introduced into the gas analyzer of 6 components present in the biomass gasification system of this example. Volume concentration and measuring result of the calibrating gas are provided in Table 3

**Table 3 Volume concentration and measuring result of the calibrating gas $CH_4$**

| Volume concentration of the calibrating gas (%) | Measured result (%) | |
|---|---|---|
| $CH_4$ | $T_{CH4}$ | $T_{CnHm}$ |
| 0 | 0 | 0 |
| 1.88 | 1.90 | 0.05 |
| 3.79 | 3.80 | 0.11 |
| 5.72 | 5.75 | 0.16 |
| 7.68 | 7.70 | 0.22 |
| 9.66 | 9.69 | 0.28 |
| 11.67 | 11.65 | 0.34 |
| 13.71 | 13.70 | 0.40 |
| 15.78 | 15.80 | 0.45 |
| 17.87 | 17.90 | 0.51 |
| 20.00 | 20.02 | 0.57 |

The following correction equation can be obtained through data analysis:

$$R_{CnHm} = T_{CnHm} - A \times T_{CH4}$$

Data in Table 3 are substituted into the equation to conclude that A = 0.02868

Therefore, $R_{CnHm} = T_{CnHm} - A \times T_{CH4} = T_{CnHm} - 0.02868 \times T_{CH4}$

2) Revise the volume concentration of $H_2$.

The balance gas $N_2$ used to calibrate $H_2$ is greatly different from $CH_4$ and $CO_2$ in relative thermal conductivity, as shown **in Table 4,** so $CH_4$ and $CO_2$ have certain influence on the measuring results of $H_2$ using TCD. $C_nH_m$ is different from $N_2$ in thermal conductivity, but its content in the coal gas is only about 1/5 as much as $CH_4$, therefore it can be neglected. CO and $O_2$ are very slightly different from $N_2$ in thermal conductivity, and can also be neglected. Hence, it is only necessary to get the revised volume concentration of $H_2$ ($R_{H2}$) through revising the measuring results of $H_2$ ($T_{H2}$) according to the measuring results of $CH_4$ and $CO_2$ ($T_{CO2}$, $T_{CH4}$).

**Table 4 Thermal conductivity of different gases**

| Gas name | Relative thermal conductivity, $\lambda/\lambda_{air}$ | Gas name | Relative thermal conductivity, $\lambda/\lambda_{air}$ |
|---|---|---|---|
| Air | 1.000 | CO | 0.964 |
| $H_2$ | 7.130 | $CO_2$ | 0.614 |
| $O_2$ | 1.015 | $SO_2$ | 0.344 |
| $N_2$ | 0.998 | $NH_3$ | 0.897 |
| He | 5.910 | $CH_4$ | 1.318 |

In order to revise the influence of $CH_4$ and $CO_2$ on the measuring result of $H_2$, the calibrating gases $CH_4$ and $CO_2$ are introduced into the gas analyzer of 6 components present in the biomass gasification system of this example.

Volume concentration and measuring result of the calibrating gases are provided in Table 5:

**Table 5 Influence of CH₄ and CO₂ on H₂**

| Volume concentration of (%) | | Measured result (%) the calibrating gas | | |
|---|---|---|---|---|
| $CH_4$ | $CO_2$ | $T_{CH4}$ | $T_{CO2}$ | $T_{H2}$ |
| 0 | 0 | 0 | 0 | 0 |
| 0 | 2.75 | 0 | 2.73 | -0.29 |
| 0 | 5.54 | 0 | 5.51 | -0.61 |
| 0 | 8.40 | 0 | 8.36 | -0.93 |
| 0 | 11.30 | 0 | 11.35 | -1.23 |
| 0 | 14.27 | 0 | 14.21 | -1.59 |
| 0 | 17.29 | 0 | 17.34 | -1.90 |
| 0 | 20.37 | 0 | 20.43 | -2.25 |
| 0 | 23.52 | 0 | 23.61 | -2.58 |
| 0 | 26.73 | 0 | 26.69 | -2.96 |
| 0 | 30.00 | 0 | 29.97 | -3.30 |
| 1.88 | 0 | 1.84 | 0 | 0.27 |
| 3.79 | 0 | 3.82 | 0 | 0.54 |
| 5.72 | 0 | 5.76 | 0 | 0.82 |
| 7.68 | 0 | 7.71 | 0 | 1.06 |
| 9.66 | 0 | 9.63 | 0 | 1.38 |
| 11.67 | 0 | 11.62 | 0 | 1.66 |
| 13.71 | 0 | 13.83 | 0 | 1.93 |
| 15.78 | 0 | 15.81 | 0 | 2.23 |
| 17.87 | 0 | 17.91 | 0 | 2.50 |
| 20 | 0 | 19.98 | 0 | 2.80 |

The following correction equation can be obtained through data analysis:

$$R_{H2} = T_{H2} - a \times T_{CH4} - b \times T_{CO2}$$

Data in Table 5 are substituted into the equation to conclude that a=0.13989; b=-0.11026

Therefore, $R_{H2} = T_{H2} - a \times (T_{CH4} + R_{CnHm}) - b \times T_{CO2} = T_{H2} - 0.13989 \times (T_{CH4} + R_{CnHm}) + 0.11026 \times T_{CO2}$.

3) Calculate the calorific value of coal gas

According to the above gas concentration, the calorific value of coal gas is obtained through substituting $T_{CO}$, $T_{CH4}$, $R_{CnHm}$ and $R_{H2}$ into the equation $Q = T_{CO} \times 12.64 + R_{H2} \times 18.79 + T_{CH4} \times 35.88 + R_{CnHm} \times 93.18$; in which, Q is expressed as MJ/m³, 12.64, 18.79, 35.88 and 93.18 are respectively the coefficient of low calorific value of CO, $H_2$, $CH_4$ and $C_nH_m$ expressed as MJ/m³.

This example is provided to design a gas analyzer of 6 components with the measuring range of CO of 40%, that of $CO_2$ of 30%, that of $CH_4$ of 20%, that of $C_nH_m$ of 5%, that of $H_2$ of 20%, and that of $O_2$ of 25%. This gas analyzer is applicable to many industries, such as air coal gasification, biomass air gasification, blast furnace, and endothermal and exothermal gas generators for heat treatment.

**Example 2 Gas analyzer for 6 components of coal gas from the biomass pyrolysis and coking**

1. Select the length and measuring range of various gas chambers

Filter in the NDIR gas detector is selected as that in Example 1. Gas chamber design: CO detector with the measuring range of 40% and CO chamber of 43 mm long; $CO_2$ detector with the measuring range of 20% and $CO_2$ chamber of 3 mm long; $CH_4$ detector with the measuring range of 50% and $CH_4$ chamber of 34 mm long; $C_nH_m$ detector

with the measuring range of 10% and $C_nH_m$ chamber of 20 mm long.

The known technique is used to make $H_2$ detector with the measuring range of 75% and $O_2$ detector with the measuring range of 25%.

2. Measure the volume concentration of various gases

CO, $CO_2$, $CH_4$ and $C_nH_m$ are measured using NDIR, and the readings are respectively expressed as $T_{CO}$, $T_{CO2}$, $T_{CH4}$ and $T_{CnHm}$.

The volume concentration of $H_2$ is measured with TCD, and is expressed as $T_{H2}$.

The volume concentration of $O_2$ is measured with ECD, and is expressed as $T_{O2}$.

3. Revise the gas volume concentration and calculate the calorific value.

1) Revise the volume concentration of $C_nH_m$ with $CH_4$.

In order to revise the influence of $CH_4$ on the measuring result of $C_nH_m$, the calibrating gas $CH_4$ is introduced into the gas analyzer of 6 components present in the biomass pyrolysis and coking system of this example. Volume concentration and measuring result of the calibrating gas are provided in Table 6:

**Table 6 Volume concentration and measuring result of the calibrating gas $CH_4$**

| Standard gas volume concentration (%) | Measuring result (%) | |
|---|---|---|
| $CH_4$ | $T_{CH4}$ | $T_{CnHm}$ |
| 0 | 0 | 0 |
| 4.29 | 4.30 | 0.12 |
| 8.72 | 8.75 | 0.25 |
| 13.3 | 13.28 | 0.38 |
| 18.02 | 18.00 | 0.51 |
| 22.91 | 22.94 | 0.65 |
| 27.96 | 28.00 | 0.78 |
| 33.18 | 33.20 | 0.94 |
| 38.59 | 38.61 | 1.11 |
| 44.19 | 44.23 | 1.26 |
| 50.00 | 50.00 | 1.41 |

The following correction equation can be obtained through data analysis:

$$R_{CnHm} = T_{CnHm} - A \times T_{CH4}$$

Data in Table 6 are substituted into the equation to conclude that A = 0.02837.

Therefore, $R_{cnHm} = T_{CnHm} - A \times T_{CH4} = T_{CnHm} - 0.02837 \times T_{CH4}$

2) Revise the volume concentration of $H_2$.

In order to revise the influence of $CH_4$ and $CO_2$ on the measuring result of $H_2$, the calibrating gases $CH_4$ and $CO_2$ are introduced into the gas analyzer of 6 components present in the biomass pyrolysis and coking system of this example. Volume concentration and measuring result of the calibrating gases are provided in Table 7:

**Table 7 Influence of $CH_4$ and $CO_2$ on $H_2$**

| Standard gas volume concentration (%) | | Measuring result (%) | | |
|---|---|---|---|---|
| $CH_4$ | $CO_2$ | $T_{CH4}$ | $T_{CO2}$ | $T_{H2}$ |
| 50 | 0 | 0 | 0 | 7.05 |
| 44.19 | 0 | 0 | 1.91 | 6.24 |
| 38.59 | 0 | 0 | 3.81 | 5.44 |

(continued)

| Standard gas volume concentration (%) | | Measuring result (%) | | |
|---|---|---|---|---|
| 33.18 | 0 | 0 | 5.71 | 4.68 |
| 27.96 | 0 | 0 | 7.66 | 3.92 |
| 22.91 | 0 | 0 | 9.62 | 3.23 |
| 18.02 | 0 | 0 | 11.61 | 2.55 |
| 13.3 | 0 | 0 | 13.75 | 1.86 |
| 8.72 | 0 | 0 | 15.81 | 1.23 |
| 4.29 | 0 | 0 | 17.91 | 0.61 |
| 0 | 20.00 | 0 | 19.99 | -2.22 |
| 0 | 17.88 | 4.32 | 0 | -1.99 |
| 0 | 15.78 | 8.71 | 0 | -1.74 |
| 0 | 13.72 | 13.28 | 0 | -1.50 |
| 0 | 11.68 | 18.04 | 0 | -1.30 |
| 0 | 9.67 | 22.93 | 0 | -1.05 |
| 0 | 7.69 | 27.92 | 0 | -0.86 |
| 0 | 5.73 | 33.15 | 0 | -0.65 |
| 0 | 3.79 | 38.62 | 0 | -0.42 |
| 0 | 1.88 | 44.23 | 0 | -0.19 |
| 0 | 0 | 50.03 | 0 | 0 |

The following correction equation can be obtained through data analysis:

$$R_{H2} = T_{H2} - a \times T_{CH4} - b \times T_{CO2}$$

Data in Table 7 are substituted into the equation to conclude that a=0.14097; b=-0.11091.

Therefore, $R_{H2} = T_{H2} - a \times (T_{CH4} + R_{CnHm)}T_{CH4} - b \times T_{CO2} = T_{H2} - 0.14097 \times (T_{CH4} + R_{CnHm)} + 0.11091 \times T_{CO2}$

3) Calculate the calorific value of coal gas

According to the above gas concentration, the calorific value of coal gas is obtained through substituting $T_{CO}$, $T_{CH4}$, $R_{CnHm}$ and $R_{H2}$ into the equation $Q = T_{CO} \times 12.64 + R_{H2} \times 18.79 + T_{CH4} \times 35.88 + R_{CnHm} \times 93.18$;

In which, Q is expressed as $MJ/m^3$, 12.64, 18.79, 35.88 and 93.18 are respectively the coefficient of low calorific value of CO, $H_2$, $CH_4$ and $C_nH_m$ expressed as $MJ/m^3$.

This example is provided to design a gas analyzer of 6 components with the measuring range of CO of 40%, that of $CO_2$ of 20%, that of $CH_4$ of 50%, that of $C_nH_m$ of 10%, that of $H_2$ of 75%, and that of $O_2$ of 25%. This gas analyzer is applicable to many industries, such as coking, biomass pyrolysis, dry distillation, and mixed gas in steel.

While particular embodiments of the invention have been shown and described, it will be obvious to those skilled in the art that changes and modifications may be made without departing from the invention in its broader aspects, and therefore, the aim in the appended claims is to cover all such changes and modifications as fall within the scope of the invention.

**Claims**

1. A method for measuring the components and calorific value of a coal gas comprising 6 components being CO, $CO_2$, $CH_4$, $C_nH_m$, $H_2$ and $O_2$ with a range of up to 40% CO, 30% $CO_2$, 20% $CH_4$, 5% $C_nH_m$, 20% $H_2$ and 25% $O_2$, comprising the steps of:

- measuring a volume concentration of $H_2$ ($T_{H2}$) in the coal gas by using a thermal conductivity detector (TCD),
- measuring a volume concentration of $O_2$ in the coal gas using an electrochemical detector (ECD), and
- calculating the calorific value of the coal gas via measured and revised volume concentrations of various gases;

the method further comprising the following steps:

1) measuring volume concentrations of CO, $CO_2$, $CH_4$, and $C_nH_m$ in the coal gas, represented by $T_{CO}$, $T_{CO2}$, $T_{CH4}$, and $T_{CnHm}$, respectively, in the coal gas using non-dispersive infrared (NDIR) technology;

2) calculating a revised volume concentration of $C_nH_m$ ($R_{CnHm}$) using the equation $R_{CnHm} = T_{CnHm}-A\times T_{CH4}$, in which $T_{CnHm}$ and $T_{CH4}$ represent the volume concentrations of $C_nH_m$ and $CH_4$ measured with NDIR, respectively, and A is 0.02868;

3) calculating a revised volume concentration of $H_2$ ($R_{H2}$) using the equation $R_{H2} = T_{H2}-a\times(T_{CH4}+R_{CnHm})-b\times T_{CO2}$, in which, $T_{H2}$ represents the measured volume concentration of $H_2$ using TCD, $T_{CH4}$ and $T_{CO2}$ represent the volume concentrations of $CH_4$ and $CO_2$ measured using NDIR, respectively, a is 0.13989 and b is -0.11026 and $R_{CnHm}$ is the revised volume concentration obtained in step 2); and

4) calculating the calorific value of the coal gas (Q) using the equation $Q = T_{CO} \times 12.64+ R_{H2} \times 18.79 + T_{CH4} \times 35.88 + R_{CnHm} \times 93.18$, in which, $T_{CO}$ and $T_{CH4}$ represent the measured volume concentrations and $R_{H2}$ and $R_{CnHm}$ represent the revised volume concentrations.

**2.** A method for measuring the components and calorific value of a coal gas comprising 6 components being CO, $CO_2$, $CH_4$, $C_nH_m$, $H_2$ and $O_2$ with a range of up to 40% CO, 20% $CO_2$, 50% $CH_4$, 10% $C_nH_m$, 75% $H_2$ and 25% $O_2$, comprising the steps of:

- measuring a volume concentration of $H_2$ ($T_{H2}$) in the coal gas by using a thermal conductivity detector (TCD),
- measuring a volume concentration of $O_2$ in the coal gas using an electrochemical detector (ECD), and
- calculating the calorific value of the coal gas via measured and revised volume concentrations of various gases;

the method further comprising the following steps:

1) measuring volume concentrations of CO, $CO_2$, $CH_4$, and $C_nH_m$ in the coal gas, represented by $T_{CO}$, $T_{CO2}$, $T_{CH4}$, and $T_{CnHm}$, respectively, in the coal gas using non-dispersive infrared (NDIR) technology;

2) calculating a revised volume concentration of $C_nH_m$ ($R_{CnHm}$) using the equation $R_{CnHm} = T_{CnHm}-A\times T_{CH4}$, in which $T_{CnHm}$ and $T_{CH4}$ represent the volume concentrations of $C_nH_m$ and $CH_4$ measured with NDIR, respectively, and A is 0.02837;

3) calculating a revised volume concentration of $H_2$ ($R_{H2}$) using the equation $R_{H2} = T_{H2}-a\times(T_{CH4}+R_{CnHm})-b\times T_{CO2}$, in which, $T_{H2}$ represents the measured volume concentration of $H_2$ using TCD, $T_{CH4}$ and $T_{CO2}$ represent the volume concentrations of $CH_4$ and $CO_2$ measured using NDIR, respectively, a is 0.14097 and b is -0.11091 and $R_{CnHm}$ is the revised volume concentration obtained in step 2); and

4) calculating the calorific value of the coal gas (Q) using the equation $Q = T_{CO} \times 12.64+ R_{H2} \times 18.79 + T_{CH4} \times 35.88 + R_{cnHm} \times 93.18$, in which, $T_{CO}$ and $T_{CH4}$ represent the measured volume concentrations and $R_{H2}$ and $R_{cnHm}$ represent the revised volume concentrations.

**3.** The method of claim 1 or 2, **characterized in that** $CH_4$ is measured using the principle of NDIR; and a selected narrowband filter parameter, that is, a center wavelength (CWL)/half-peak bandwidth (HWBP), is $7.85\pm0.05\mu m$ / $180\pm5$ nm.

**4.** The method of claim 1 or 2, **characterized in that** $C_nH_m$ is measured using the principle of NDIR; and a selected narrowband filter parameter, that is, a CWL/HWBP, is $3.46 \pm 0.05\mu m$ / $120 \pm 5$ nm.

**5.** The method of claim 1 or 2, **characterized in that** CO is measured using the principle of NDIR; and a selected narrowband filter parameter, that is, a CWL/HWBP, is $4.66 \pm 0.05\mu m$ / $90 \pm 5$ nm.

**6.** The method of claim 1 or 2, **characterized in that** $CO_2$ is measured using the principle of NDIR; and a selected narrowband filter parameter, that is, a CWL/HWBP, is $4.26 \pm 0.05\mu m$ / $120 \pm 5$ nm.

**Patentansprüche**

**1.** Verfahren zum Messen der Komponenten und des Heizwerts eines Kohlegases umfassend 6 Komponenten, die CO, $CO_2$, $CH_4$, $C_nH_m$, $H_2$ und $O_2$ sind, mit einer Verteilung von bis zu 40 % CO, 30 % $CO_2$, 20 % $CH_4$, 5 % $C_nH_m$, 20 % $H_2$ und 25 % $O_2$, umfassend die Schritte des:

- Messens einer Volumenkonzentration von $H_2$ ($T_{H2}$) in dem Kohlegas unter Anwendung eines Wärmeleitfähigkeitsdetektors (TCD),
- Messens einer Volumenkonzentration von $O_2$ in dem Kohlegas unter Anwendung eines elektrochemischen Detektors (ECD) und
- Berechnens des Heizwerts des Kohlegases durch gemessene und revidierte Volumenkonzentrationen verschiedener Gase;

wobei das Verfahren ferner die folgenden Schritte umfasst:

1) Messen von Volumenkonzentrationen von CO, $CO_2$, $CH_4$ und $C_nH_m$ in dem Kohlegas, die jeweils durch Tco, $T_{CO2}$, $T_{CH4}$ und $T_{CnHm}$ dargestellt sind, in dem Kohlegas unter Anwendung nichtdispersiver Infrarot-(NDIR) Technologie;
2) Berechnen einer revidierten Volumenkonzentration von $C_nH_m$ ($R_{CnHm}$) unter Anwendung der Gleichung $R_{CnHm} = T_{CnHm} - A \times T_{CH4}$, wobei $T_{CnHm}$ und $T_{CH4}$ jeweils die Volumenkonzentrationen von $C_nH_m$ und $CH_4$, mit NDIR gemessen, darstellen und A den Wert 0,02868 hat;
3) Berechnen einer revidierten Volumenkonzentration von $H_2$ ($R_{H2}$) unter Anwendung der Gleichung $R_{H2} = T_{H2} - a \times (T_{CH4} + R_{CnHm}) - b \times T_{CO2}$, wobei $T_{H2}$ die gemessene Volumenkonzentration von $H_2$ unter Anwendung von Wäremeleitfähigkeitsdetektion (TCD) darstellt, $T_{CH4}$ und $T_{CO2}$ jeweils die Volumenkonzentration von $CH_4$ und $CO_2$, mittels NDIR gemessen, darstellen, a den Wert 0,13989 hat und b den Wert -0,11026 hat und $R_{cnHm}$ die revidierte, in Schritt 2) erhaltene Volumenkonzentration ist; und
4) Berechnen des Heizwerts des Kohlegases (Q) unter Anwendung der Gleichung $Q = Tco \times 12,64 + R_{H2} \times 18,79 + T_{CH4} \times 35,88 + R_{CnHm} \times 93,18$, wobei $T_{CO}$ und $T_{CH4}$ die gemessenen Volumenkonzentrationen darstellen und $R_{H2}$ und $R_{CnHm}$ die revidierten Volumenkonzentrationen darstellen.

2. Verfahren zum Messen der Komponenten und des Heizwerts eines Kohlegases umfassend 6 Komponenten, die CO, $CO_2$, $CH_4$, $C_nH_m$, $H_2$ und $O_2$ sind, mit einer Verteilung von bis zu 40 % CO, 20 % $CO_2$, 50 % $CH_4$, 10 % $C_nH_m$, 75 % $H_2$ und 25 % $O_2$, umfassend die Schritte des:

- Messens einer Volumenkonzentration von $H_2$ ($T_{H2}$) in dem Kohlegas unter Anwendung eines Wärmeleitfähigkeitsdetektors (TCD),
- Messens einer Volumenkonzentration von $O_2$ in dem Kohlegas unter Anwendung eines elektrochemischen Detektors (ECD) und
- Berechnens des Heizwerts des Kohlegases durch gemessene und revidierte Volumenkonzentrationen verschiedener Gase;

wobei das Verfahren ferner die folgenden Schritte umfasst:

1) Messen von Volumenkonzentrationen von CO, $CO_2$, $CH_4$ und $C_nH_m$ in dem Kohlegas, die jeweils durch Tco, $T_{CO2}$, $T_{CH4}$ und $T_{CnHm}$ dargestellt sind, in dem Kohlegas unter Anwendung nichtdispersiver Infrarot-(NDIR) Technologie;
2) Berechnen einer revidierten Volumenkonzentration von $C_nH_m$ ($R_{CnHm}$) unter Anwendung der Gleichung $R_{CnHm} = T_{CnHm} - A \times T_{CH4}$, wobei $T_{CnHm}$ und $T_{CH4}$ jeweils die Volumenkonzentrationen von $C_nH_m$ und $CH_4$, mit NDIR gemessen, darstellen und A den Wert 0,02837 hat;
3) Berechnen einer revidierten Volumenkonzentration von $H_2$ ($R_{H2}$) unter Anwendung der Gleichung $R_{H2} = T_{H2} - a \times (T_{CH4} + R_{CnHm}) - b \times T_{CO2}$, wobei $T_{H2}$ die gemessene Volumenkonzentration von $H_2$ unter Anwendung von Wärmeleitfähigkeitsdetektion (TCD) darstellt, $T_{CH4}$ und $T_{CO2}$ jeweils die Volumenkonzentrationen von $CH_4$ und $CO_2$, mittels NDIR gemessen, darstellen, a den Wert 0,14097 hat und b den Wert -0,11091 hat und $R_{CnHm}$ die revidierte, in Schritt 2) erhaltene Volumenkonzentration ist; und
4) Berechnen des Heizwerts des Kohlegases (Q) unter Anwendung der Gleichung $Q = T_{CO} \times 12,64 + R_{H2} \times 18,79 + T_{CH4} \times 35,88 + R_{CnHm} \times 93,18$, wobei $T_{CO}$ und $T_{CH4}$ die gemessenen Volumenkonzentrationen darstellen und $R_{H2}$ und $R_{CnHm}$ die revidierten Volumenkonzentrationen darstellen.

3. Verfahren nach Anspruch 1 oder 2, **dadurch gekennzeichnet, dass** $CH_4$ unter Anwendung des Prinzips von NDIR gemessen wird; und ein ausgewählter Schmalbandfilterparameter, das heißt eine mittlere Wellenlänge (CWL)/Halb-Peak-Bandbreite (HWBP) 7,85±0,05 $\mu$m/180 ± 5 nm beträgt.

4. Verfahren nach Anspruch 1 oder 2, **dadurch gekennzeichnet, dass** $C_nH_m$ unter Anwendung des Prinzips von NDIR gemessen wird; und ein ausgewählter Schmalbandfilterparameter, das heißt eine CWL/HWBP, 3,46±0,05

μm/120 ± 5 nm beträgt.

**5.** Verfahren nach Anspruch 1 oder 2, **dadurch gekennzeichnet, dass** CO unter Anwendung des Prinzips von NDIR gemessen wird; und ein ausgewählter Schmalbandfilterparameter, das heißt eine CWL/HWBP, 4,66±0,05 μm/90 ± 5 nm beträgt.

**6.** Verfahren nach Anspruch 1 oder 2, **dadurch gekennzeichnet, dass** $CO_2$ unter Anwendung des Prinzips von NDIR gemessen wird; und ein ausgewählter Schmalbandfilterparameter, das heißt eine CWL/HWBP, 4,26 ± 0,05 μm/120 + 5 nm beträgt.

**Revendications**

**1.** Méthode de mesure des composants et de la valeur calorifique d'un gaz de houille comprenant 6 composants qui sont CO, $CO_2$, $CH_4$, $C_nH_m$, $H_2$ et $O_2$ avec une plage allant jusqu'à 40 % de CO, 30 % de $CO_2$, 20 % de $CH_4$, 5 % de $C_nH_m$, 20 % d'$H_2$ et 25 % d'$O_2$, comprenant les étapes consistant à :

- mesurer une concentration volumique d'$H_2$ ($T_{H2}$) dans le gaz de houille en utilisant un détecteur à conductivité thermique (TCD),
- mesurer une concentration volumique d'$O_2$ dans le gaz de houille en utilisant un détecteur électrochimique (ECD), et
- calculer la valeur calorifique du gaz de houille par l'intermédiaire des concentrations volumiques mesurées et révisées des divers gaz ;

la méthode comprenant en outre les étapes suivantes consistant à :

1) mesurer les concentrations volumiques de CO, $CO_2$, $CH_4$ et $C_nH_m$ dans le gaz de houille, représentées respectivement par Tco, $T_{CO2}$, $T_{CH4}$ et $T_{CnHm}$, dans le gaz de houille en utilisant une technologie infrarouge non dispersive (NDIR) ;
2) calculer une concentration volumique révisée de $C_nH_m$ ($R_{CnHm}$) en utilisant l'équation $R_{CnHm} = T_{CnHm} - A \times T_{CH4}$, dans laquelle $T_{CnHm}$ et $T_{CH4}$ représentent respectivement les concentrations volumiques de $C_nH_m$ et $CH_4$ mesurées avec la NDIR, et A représente 0,02868 ;
3) calculer une concentration volumique révisée d'$H_2$ ($R_{H2}$) en utilisant l'équation $R_{H2} = T_{H2} - a \times (T_{CH4} + R_{CnHm}) - b \times T_{CO2}$, dans laquelle $T_{H2}$ représente la concentration volumique mesurée d'$H_2$ en utilisant le TCD, $T_{CH4}$ et $T_{CO2}$ représentent respectivement les concentrations volumiques de $CH_4$ et $CO_2$ mesurées en utilisant la NDIR, a représente 0,13989 et b représente -0,11026 et $R_{CnHm}$ représente la concentration volumique révisée obtenue à l'étape 2) ; et
4) calculer la valeur calorifique du gaz de houille (Q) en utilisant l'équation $Q = T_{CO} \times 12,64 + R_{H2} \times 18,79 + T_{CH4} \times 35,88 + R_{CnHm} \times 93,18$, dans laquelle Tco et $T_{CH4}$ représentent les concentrations volumiques mesurées et $R_{H2}$ et $R_{CnHm}$ représentent les concentrations volumiques révisées.

**2.** Méthode de mesure des composants et de la valeur calorifique d'un gaz de houille comprenant 6 composants qui sont CO, $CO_2$, $CH_4$, $C_nH_m$, $H_2$ et $O_2$ avec une plage allant jusqu'à 40 % de CO, 20 % de $CO_2$, 50 % de $CH_4$, 10 % de $C_nH_m$, 75 % d'$H_2$ et 25 % d'$O_2$, comprenant les étapes consistant à :

- mesurer une concentration volumique d'$H_2$ ($T_{H2}$) dans le gaz de houille en utilisant un détecteur à conductivité thermique (TCD),
- mesurer une concentration volumique d'$O_2$ dans le gaz de houille en utilisant un détecteur électrochimique (ECD), et
- calculer la valeur calorifique du gaz de houille par l'intermédiaire des concentrations volumiques mesurées et révisées des divers gaz ;

la méthode comprenant en outre les étapes suivantes consistant à :

1) mesurer les concentrations volumiques de CO, $CO_2$, $CH_4$ et $C_nH_m$ dans le gaz de houille, représentées respectivement par Tco, $T_{CO2}$, $T_{CH4}$ et $T_{CnHm}$, dans le gaz de houille en utilisant une technologie infrarouge non dispersive (NDIR) ;
2) calculer une concentration volumique révisée de $C_nH_m$ ($R_{CnHm}$) en utilisant l'équation $R_{CnHm} = T_{CnHm} - A \times$

$T_{CH4}$, dans laquelle $T_{CnHm}$ et $T_{CH4}$ représentent respectivement les concentrations volumiques de $C_nH_m$ et $CH_4$ mesurées avec la NDIR, et A représente 0,02837 ;

3) calculer une concentration volumique révisée d'$H_2$ ($R_{H2}$) en utilisant l'équation $R_{H2} = T_{H2} - a \times (T_{CH4} + R_{CnHm}) - b \times T_{CO2}$, dans laquelle $T_{H2}$ représente la concentration volumique mesurées d'$H_2$ en utilisant le TCD, $T_{CH4}$ et $T_{CO2}$ représentent respectivement les concentrations volumiques de $CH_4$ et $CO_2$ mesurées en utilisant la NDIR, a représente 0,14097 et b représente -0,11091 et $R_{CnHm}$ représente la concentration volumique révisée obtenue à l'étape 2) ; et

4) calculer la valeur calorifique du gaz de houille (Q) en utilisant l'équation $Q = T_{CO} \times 12,64 + R_{H2} \times 18,79 + T_{CH4} \times 35,88 + R_{CnHm} \times 93,18$, dans laquelle Tco et $T_{CH4}$ représentent les concentrations volumiques mesurées et $R_{H2}$ et $R_{CnHm}$ représentent les concentrations volumiques révisées.

3. Méthode selon la revendication 1 ou 2, **caractérisée en ce que** $CH_4$ est mesuré en utilisant le principe de NDIR ; et un paramètre de filtre à bande étroite sélectionné, c'est-à-dire une longueur d'onde centrale (CWL)/largeur de bande à mi-hauteur de pic (HWBP), est $7,85 \pm 0,05$ $\mu$m / $180 \pm 5$ nm.

4. Méthode selon la revendication 1 ou 2, **caractérisée en ce que** $C_nH_m$ est mesuré en utilisant le principe de NDIR ; et un paramètre de filtre à bande étroite sélectionné, c'est-à-dire CWL/HWBP, est $3,46 \pm 0,05$ $\mu$m / $120 \pm 5$ nm.

5. Méthode selon la revendication 1 ou 2, **caractérisée en ce que** CO est mesuré en utilisant le principe de NDIR ; et un paramètre de filtre à bande étroite sélectionné, c'est-à-dire CWL/HWBP, est $4,66 \pm 0,05$ $\mu$m / $90 \pm 5$ nm.

6. Méthode selon la revendication 1 ou 2, **caractérisée en ce que** $CO_2$ est mesuré en utilisant le principe de NDIR ; et un paramètre de filtre à bande étroite sélectionné, c'est-à-dire CWL/HWBP, est $4,26 \pm 0,05$ $\mu$m / $120 \pm 5$ nm.

**FIG. 1**

**FIG. 2**

**FIG. 3**

**FIG. 4**

16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201886002 U
- CN 101750439 A
- US 2008011952 A1

- DE 102005005727 A1
- DE 102008029553 B3